# FASCICULE DE BREVET EUROPEEN

(11) **EP 2 480 872 B1**
(45) Date de publication et mention de la délivrance du brevet: **07.08.2013**
(21) Numéro de dépôt: 10757415.4
(22) Date de dépôt: 24.09.2010
(51) Int. Cl.: G01N 1/14

(54) **DISPOSITIF DE PRELEVEMENT D'ECHANTILLONS LIQUIDES HORS D'UNE CUVE**
VORRICHTUNG ZUR ENTNAHME VON FLÜSSIGPROBEN AUS EINEM TANK
DEVICE FOR COLLECTING LIQUID SAMPLES FROM A VAT

(30) Priorité: 25.09.2009 FR 0956644
(43) Date de publication de la demande: 01.08.2012
(73) Titulaire: Commissariat à l'Énergie Atomique et aux Énergies Alternatives, 75015 Paris (FR)
(72) Inventeur: BRENNEIS, Christophe, F-30290 Saint Victor La Coste (FR); LAGRAVE, Hervé, 30290 Laudun (FR); MAMERT, Jacques, F-91250 Saint Germain Les Corbeil (FR)
(74) Mandataire: Ilgart, Jean-Christophe
(86) Numéro de dépôt international: PCT/EP2010/064161
(87) Numéro de publication internationale: WO 2011/036257

(56) Documents cités:
- EP-A1- 0 063 971
- EP-A1- 0 296 917
- EP-A2- 0 155 107
- US-A- 4 674 343
- US-A- 5 353 652

## Description

Le sujet de l'invention est un dispositif de prélèvement d'échantillons liquides hors d'une cuve, voire d'une pluralité de cuves.

De tels dispositifs sont courants dans l'industrie nucléaire afin d'analyser certains liquides chargés en matières irradiantes. Les dispositifs correspondants sont généralement placés dans des cellules blindées, et on agit sur eux par des télémanipulateurs. Le liquide à analyser est d'abord présent dans une cuve. Il subit une aspiration par un conduit débouchant dans le sommet de la cuve, qui l'achemine vers un support de prélèvement sur lequel un petit récipient, appelé généralement cruchon, a été posé, et il emplit partiellement ce cruchon. Le surplus de liquide est évacué vers un exutoire ou revient dans la cuve en s'écoulant par le conduit, qui est incliné, dès que l'aspiration a cessé.

Un inconvénient de ces dispositifs est que le retour à la cuve n'est pas complet et que des gouttelettes de liquide demeurent sur le conduit, puis se cristallisent: cela peut être considéré comme polluant, et limite la qualité des mesures suivantes en se mêlant aux échantillons aspirés ensuite quand le liquide d'origine de la cuve a été remplacé par un autre. Un autre inconvénient de ces dispositifs est qu'il est difficile de les mettre en commun à une batterie de cuves pour prélever au choix des échantillons de l'une quelconque d'elles.

L'art antérieur proche comprend encore les documents US-A-5 353 652, US-A-4 674 343 et EP-A-0 063 971.

L'invention a pour objet d'éliminer ces inconvénients, et avant tout de permettre un nettoyage suffisant du conduit et des conduits menant aux cuves sans compliquer grandement le dispositif ni lui faire perdre sa faculté de pouvoir être actionné par télémanipulation.

Sous une forme générale, elle concerne un dispositif de prélèvement d'échantillons liquides hors d'au moins une cuve, comprenant au moins un conduit se raccordant à une cuve respective et aboutissant à un exutoire distinct de la cuve, un support de cruchons de prélèvement des échantillons disposé sur le conduit, et une unité d'aspiration du contenu de la cuve dans le conduit, l'unité d'aspiration comprenant une buse disposée sur le conduit, une arrivée d'air comprimé débouchant dans la buse, caractérisé en ce que le dispositif comprend encore une vanne d'obturation du conduit, disposée sur le conduit entre la buse et l'exutoire, et l'exutoire est à une extrémité du conduit qui est opposée à la cuve.

Cette disposition permet d'utiliser l'air comprimé pour produire l'aspiration en se détendant dans la buse, puis, dès que la vanne est fermée, pour nettoyer le conduit en inversant sa circulation vers la cuve, ce qui rejette les gouttelettes du liquide qui y séjournent encore.

Le conduit comprend avantageusement un petit réservoir et le support de cruchons de prélèvement est apte à être connecté sur le petit réservoir par l'intermédiare d'un raccord rapide auto-obturant en deux parties, une première desdites parties appartenant au petit réservoir et une seconde desdites parties comprenant une aiguille qui pénètre à l'intérieur du petit réservoir en traversant la première partie: cette disposition permet de disposer d'un support de cruchon facilement maniable par télémanipulation pour être installé sur le dispositif de prélèvement, puis retiré pour enlever le cruchon et le remplacer sans difficulté à l'écart du dispositif.

Le conduit est avantageusement plus large du côté de la buse qui mène de l'exutoire que vers le côté opposé, qui mène vers la cuve. Ce dispositif facilite la bonne direction de l'air comprimé quand il arrive à la buse, vers l'exutoire quand la vanne est ouverte, grâce à la résistance plus faible qu'il rencontre de ce côté.

Afin de faciliter l'entretien du dispositif alors que seules des télémanipulations sont possibles, ont préconise que la buse et la vanne forment un ensemble unitaire et amovible avec une portion du conduit et une portion de l'arrivée d'air comprimé, lesdites portions étant raccordées à des portions principales du conduit et de l'arrivée d'air comprimé par des raccords à cabestan.

Selon un perfectionnement, le dispositif comprend une pluralité de cuves et de conduits, les conduits se joignant les uns aux autres à un sélecteur, et comprenant ensuite une portion commune vers l'exutoire, sur laquelle la buse et la vanne sont disposées.

Le dispositif peut alors desservir plusieurs cuves et plusieurs conduits. Une réalisation favorable comprend un sélecteur tournant qui comprend un élément mobile cylindrique muni d'un organe externe de manoeuvre, l'élément mobile comprenant un perçage axial dirigé vers l'exutoire et un perçage radial dirigé vers les cuves et des raccordements des conduits disposés sur une portion de tour de sélecteur.

L'invention sera maintenant décrite plus complètement et à titre illustratif en liaison aux figures.

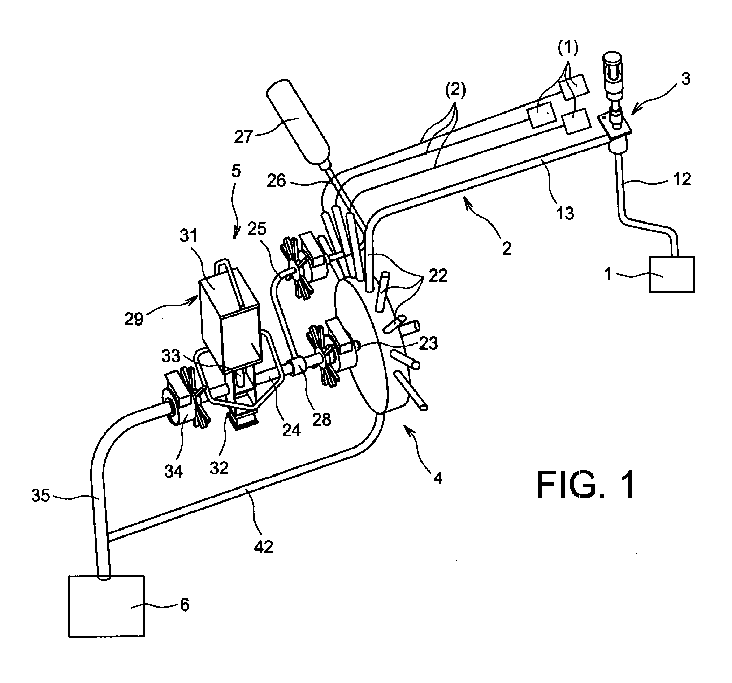

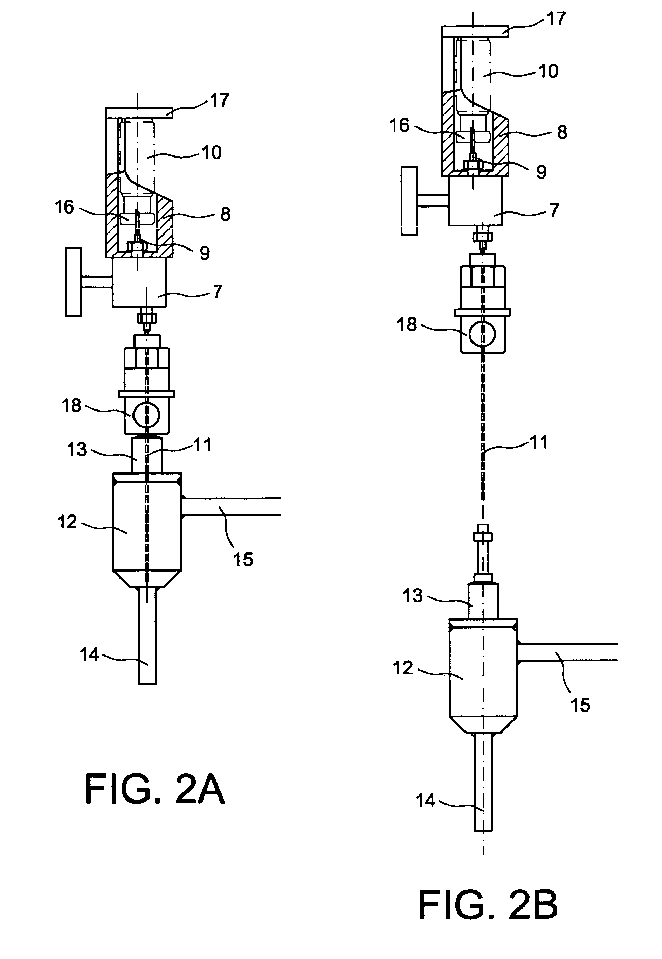

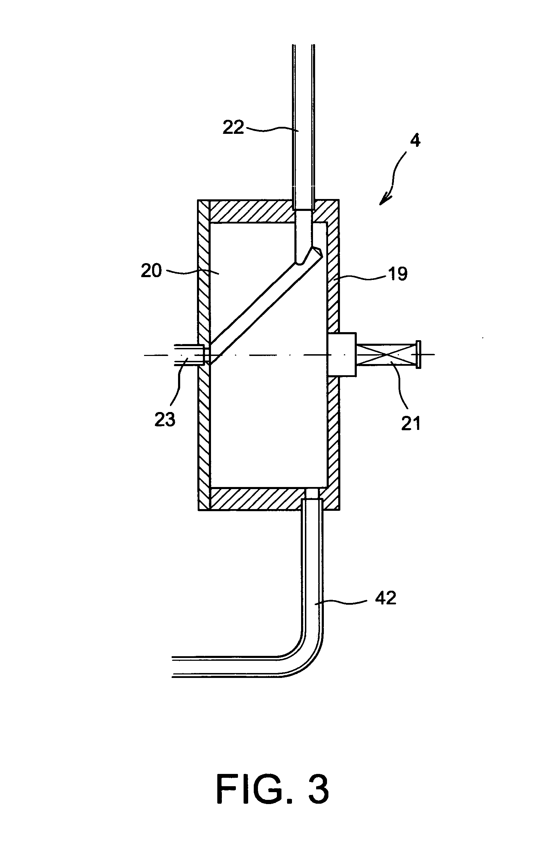

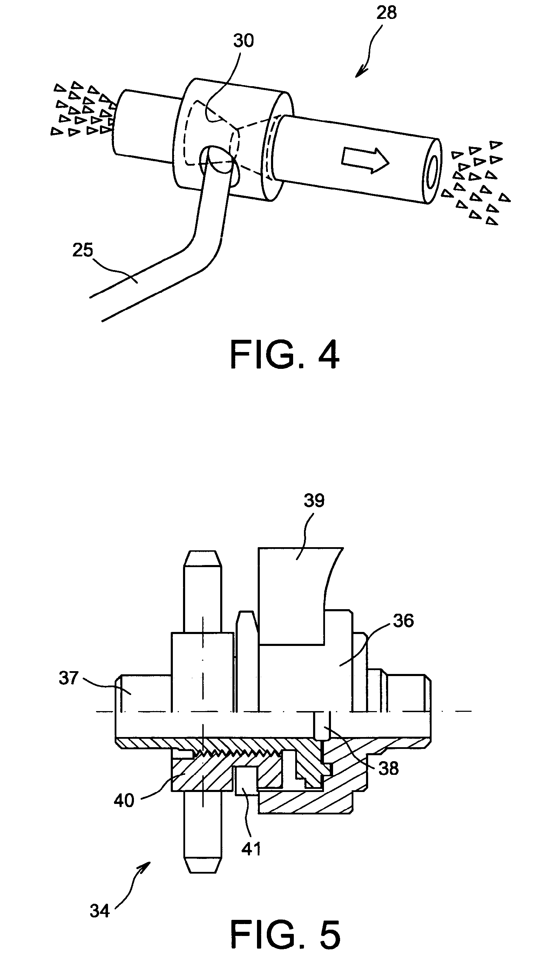

La figure 1 est une vue générale de l'invention, la figure 2 une vue de l'ensemble de prélèvement (2A en assemblé, 2B en éclaté), la figure 3 une vue du sélecteur, la figure 4 une vue de la buse et la figure 5 une vue d'un raccord à cabestan.

On se reporte d'abord à la figure 1. Le liquide à prélever se trouve à l'origine dans des cuves 1 de stockage. Des conduits 2 de prélèvement débouchent au sommet des cuves 1, passent par une installation de prélèvement 3, respective, se joignent à un sélecteur 4 commun, puis passent par une unité d'aspiration 5, et finissent vers un exutoire 6 qui est un réservoir de collecte du liquide aspiré en surplus. Les éléments principaux du dispositif seront maintenant décrits successivement. Les conduits 2 s'élèvent d'abord à partir des cuves 1, jusqu'aux installations de prélèvement 3, puis s'abaissent peu à peu vers l'exutoire 6, opposé aux cuves 1 et donc distinct d'elles.

Le système de prélèvement 3 apparaît en détail à la figure 2. Il comprend d'abord une partie mobile constituée d'une vanne manuelle 7 dont le sommet est occupé par un étui 8 cylindrique ouvert en haut comprenant une aiguille supérieure 9 sur laquelle pourra être mis en place le cruchon 10 et une partie pivotante 17 permettant d'emprisonner le cruchon 10 dans son logement, et la base par une aiguille 11 sur laquelle est fixé un raccord rapide auto-obturant femelle 18 et par laquelle le prélèvement sera effectué. Dans le cas d'une pluralité de cuves à prélever, cette partie mobile est déplacée et munie d'un nouveau cruchon.
L'ensemble comprend aussi une partie fixe comprenant un petit réservoir 12 clos par un raccord rapide auto-obturant mâle 13 et dans lequel débouchent une portée inférieure 14 du conduit 2 menant à la cuve 1, une deuxième portée 15 du conduit 2 menant au sélecteur 4. Pour réaliser un prélèvement, la partie mobile est installée sur la partie fixe, par insertion de l'aiguille 11 dans le raccord 13 jusqu'à connexion du raccord rapide femelle 18 sur le raccord rapide mâle 13. Un cruchon 10 neuf a été préalablement installé dans la partie mobile sur l'aiguille 9, la vanne manuelle 7 étant en position fermée. Ces cruchons sont hermétiques et le vide y est réalisé au départ. Leur septum 16 est en caoutchouc et peut être percé par l'aiguille 9 quand les cruchons 10 sont enfoncés sur elle. Cela est fait quand une aspiration a permis d'emplir le petit réservoir 12 du liquide à prélever : la vanne manuelle 7 est ouverte et le vide régnant dans le cruchon 10 aspire alors une partie du liquide par l'aiguille 11, la vanne manuelle 7 et l'aiguille 9. Après quoi, la vanne manuelle 7 peut être fermée, et la partie mobile peut être retirée par déconnexion au niveau des raccords rapides 13 et 18. Le cruchon 10 peut être retiré, et le perçage réalisé dans le septum 16 se referme de lui-même.

On repasse à la figure 1, et à la figure 3, pour la description du sélecteur 4. Il comprend un boisseau 19 occupé par un tournant 20 cylindrique, muni d'un organe de manoeuvre sous forme d'un bout d'arbre carré 21 saillant hors du boisseau 19. Au boisseau 19 on branche des raccords 22 prolongeant les deuxièmes portées 13 des conduits 2 et qui s'étendent en direction rayonnante du boisseau 19 sur une portion de tour. Un autre raccord 23 s'embranche au boisseau 19, mais cette fois en direction axiale, à l'opposé du bout d'arbre carré 21. Ce raccord 23 est relié à l'unité d'aspiration 5. Le tournant 20 contient un perçage 43 menant au raccord 23 par une portion axiale et, d'après la rotation du bout d'arbre carré 21, à l'un ou l'autre des raccords 22 par une portion radiale. Le sélecteur 4 permet de choisir la cuve 1 d'où le liquide est prélevé, d'après sa manoeuvre et au moyen des dispositions décrites maintenant.

L'unité d'aspiration 5 comprend une portion amovible 24 du conduit 2, une portion amovible 25 d'une arrivée d'air comprimé 26 jointe à une source d'air comprimé 27, et, installées sur la portion amovible 24, une buse 28 et une vanne 29. La buse 28, représentée à la figure 4, comprend un venturi 30 et la portion amovible 25 de l'arrivée d'air comprimé 26 y débouche. L'air comprimé se détendant dans le venturi 30 acquiert une vitesse supersonique apte à réaliser l'aspiration du liquide par le conduit 2. La vanne 29 comprend un actionneur 31 s'étendant au-dessus de la portion amovible 24 et commandant un clapet 32 pour ouvrir ou fermer la portion amovible 24 par l'intermédiaire d'une tige 33.

L'unité d'aspiration 5 peut être retirée du reste du dispositif puis remise en place, étant unie par trois raccords à cabestan 34 à l'arrivée d'air comprimé 26, au raccord 23 en sortie du sélecteur 4 et à une troisième portée 35 du conduit 2 après la vanne 29 qui mène à l'exutoire 6. Les raccords à cabestan 34, représentés plus complètement à la figure 5, comprennent un boîtier 36 et un embout 37 à assembler l'un à l'autre, l'étanchéité étant réalisée par un joint biconique 38 placé entre eux. Le boîtier 36 porte un réceptacle 39 en forme d'auge recevant et maintenant l'extrémité de l'embout 37. De plus, un écrou à cabestan 40, engagé sur un filetage extérieur de l'embout 37, est aussi maintenu par une lèvre 41 extérieure du réceptacle 39. Une rotation appliquée à l'écrou à cabestan 40 déplace l'embout 37 axialement dans le réceptacle 39 et le comprime contre le boîtier 36 en écrasant le joint biconique 38.

On a encore représenté un conduit d'évacuation 42 reliant l'intérieur du boisseau 19 à l'exutoire 6, et destiné à l'évacuation de l'humidité dans le sélecteur 4, aux figures 1 et 3.

Quand l'air comprimé est donné, la vanne 29 étant ouverte, le liquide est aspiré hors de la cuve 1 choisie vers l'unité d'aspiration 5 et l'exutoire 6 en emplissant le petit réservoir 12 où il peut être prélevé. On remarque que l'arrivée d'air débouche perpendiculairement dans le venturi 30, mais un bon sens de circulation de l'air est garanti en choisissant les portées 14, 15 et 35 du conduit 2 avec des diamètres qui s'élargissent successivement de la cuve 1 à l'exutoire 6 (sans considérer les diamètres à la buse 28), qui peut aussi être en dépression par rapport à la cuve 1 : la résistance à l'écoulement de l'air comprimé est donc moins grande vers l'exutoire 6. Mais quand l'aspiration cesse, la fermeture de la vanne 29 a pour effet de fermer la portion amovible 24 et d'inverser le sens d'écoulement de l'air comprimé vers la cuve 1, en y refoulant aussi le liquide qui aurait pu stagner dans le dispositif et notamment dans le petit réservoir 12 et le conduit 2. Aucune autre manoeuvre n'est nécessaire.

Toutes ces manoeuvres sont faciles à accomplir par des engins de télémanipulation ordinaires, à la dextérité limitée.

## Revendications

1. Dispositif de prélèvement d'échantillons liquides hors d'au moins une cuve (1), comprenant au moins un conduit (2) se raccordant à une cuve respective et aboutissant à un exutoire (6) à une extrémité opposée à la cuve, un support (8) de cruchons (10) de prélèvement des échantillons disposé sur le conduit, et une unité d'aspiration du contenu de la cuve dans le conduit, **caractérisé en ce que** l'unité d'aspiration comprend une buse (28) disposée sur le conduit, une arrivée d'air comprimé (26) débouchant dans la buse, et le dispositif comprend encore une vanne (29) d'obturation du conduit, disposée sur le conduit entre la buse (28) et l'exutoire (6).

2. Dispositif de prélèvement d'échantillons liquides selon la revendication 1, **caractérisé en ce que** le conduit (2) est plus large d'un côté de la buse (28) vers l'exutoire (6) que vers un côté de la buse vers la cuve.

3. Dispositif de prélèvement d'échantillons liquides selon la revendication 1 ou 2, **caractérisé en ce que** la buse et la vanne forment un ensemble unitaire et amovible avec une portion amovible (24) du conduit (2) et une portion amovible (25) de l'arrivée d'air comprimé (26), lesdites portions amovibles étant raccordées à des portions principales du conduit et de l'arrivée d'air comprimé par des raccords à cabestan (34).

4. Dispositif de prélèvement d'échantillons liquides selon l'une quelconque des revendications 1 à 3, **caractérisé en ce qu'**il comprend une pluralité de cuves (1) et de conduits (2), les conduits se joignant les uns aux autres à un sélecteur (4), et comprenant ensuite une portion commune vers l'exutoire (6), sur laquelle la buse (28) et la vanne (29) sont disposées.

5. Dispositif de prélèvement d'échantillons liquides selon la revendication 4, **caractérisé en ce que** le sélecteur est tournant et comprend un élément mobile cylindrique (20) muni d'un organe externe (21) de manoeuvre, l'élément mobile comprenant un perçage axial dirigé vers l'exutoire et un perçage radial dirigé vers les cuves et des raccords (22) des conduits disposés sur une portion de tour de sélecteur.

6. Dispositif de prélèvement d'échantillons liquides selon l'une quelconque des revendications 1 à 5, **caractérisé en ce que** le conduit (2) comprend un petit réservoir (12), et le support (8) de cruchons de prélèvement est apte à être connecté sur le petit réservoir (12) par l'intermédiaire d'un raccord rapide auto-obturant en deux parties (13, 18), une première desdites parties appartenant au petit réservoir et une seconde desdites parties comprenant une aiguille (11) qui pénètre à l'intérieur du petit réservoir (12) en traversant la première partie.

## Patentansprüche

1. Vorrichtung zur Entnahme von Flüssigkeitsproben aus wenigstens einem Behälter (1), umfassend wenigstens eine Leitung (2), welche mit einem zugehörigen Behälter verbunden ist und in einen Auslass (6) an einem dem Behälter gegenüberliegenden Ende mündet, ein Trägerelement (8) für Gefäße (10) zum Entnehmen von Proben, welches sich in der Leitung befindet, und eine Einheit zum Ansaugen des Inhalts des Behälters in die Leitung,
**dadurch gekennzeichnet, dass** die Ansaugeinheit eine Düse (28) umfasst, welche an der Leitung angeordnet ist, wobei ein Einlass für komprimierte Luft (26) in die Düse mündet, und dass die Vorrichtung ein Ventil (29) zum Verschließen der Leitung umfasst, welches an der Leitung zwischen der Düse (28) und dem Auslass (6) angeordnet ist.

2. Vorrichtung zur Entnahme von Flüssigkeitsproben nach Anspruch 1,
**dadurch gekennzeichnet, dass** die Leitung (2) Abfluss (6)-seitig der Düse (28) größer ist als Behälter-seitig der Düse.

3. Vorrichtung zur Entnahme von Flüssigkeitsproben nach Anspruch 1 oder 2,
**dadurch gekennzeichnet, dass** die Düse und das Ventil eine einheitliche und herausnehmbare Baugruppe bilden, zusammen mit einem herausnehmbaren Abschnitt (24) der Leitung (2) und einem herausnehmbaren Abschnitt (25) des Einlasses für komprimierte Luft (26), wobei die herausnehmbaren Abschnitte an Hauptabschnitten der Leitung und des Einlasses für komprimierte Luft mittels Winden/Spillverbindungen (34) befestigt sind.

4. Vorrichtung zur Entnahme von Flüssigkeitsproben nach einem der Ansprüche 1 bis 3,
**dadurch gekennzeichnet, dass** sie eine Mehrzahl von Behältern (1) und Leitungen (2) umfasst, wobei die Leitungen an einem Umschalter (4) miteinander verbunden sind, und daran anschließend einen gemeinsamen Abschnitt in Richtung des Auslasses (6) umfassen, an welchem die Düse (28) und das Ventil (29) angeordnet sind.

5. Vorrichtung zur Entnahme von Flüssigkeitsproben nach Anspruch 4,
**dadurch gekennzeichnet, dass** der Umschalter drehbar ist und ein bewegliches zylindrisches Element (20) umfasst, welches mit einer externen Antriebsvorrichtung (21) bereitgestellt ist, wobei das bewegliche Element eine axiale Bohrung, welche in Richtung des Auslasses gerichtet ist, und eine radiale Bohrung, welche in Richtung der Behälter gerichtet ist, und Anschlüsse (22) der Leitungen umfasst, welche an einem Abschnitt des Umfangs des Umschalters angeordnet sind.

6. Vorrichtung zur Entnahme von Flüssigkeitsproben nach einem der Ansprüche 1 bis 5,
**dadurch gekennzeichnet, dass** die Leitung (2) ein kleines Reservoir (12) umfasst, und das Trägerelement (8) für Gefäße zur Entnahme an das kleine Reservoir (12) über eine zweiteilige selbstschließende Schnell-Verbindung (13, 18) angeschlossen werden kann, wobei ein erstes von diesen Teilen dem kleinen Reservoir zugeordnet ist, und ein zweites von diesen Teilen eine Nadel (11) umfasst, welche in das Innere des kleinen Reservoirs (12) eindringt, indem sie durch das erste Teil hindurchgeht.

## Claims

1. Device for collecting liquid samples from at least one vat (1), comprising at least one pipe (2) connecting to a respective vat and exiting at an outlet (6) at an end opposite the vat, a samplingjug holder (8) arranged on the pipe for jugs (10) sampling the samples, and a suction unit of the contents of the vat in the pipe, **characterised in that** the suction unit comprises a nozzle (28) arranged on the pipe, a compressed air inlet (26) exiting in the nozzle, and the device further comprises a valve (29) for sealing off the pipe, arranged on the pipe between the nozzle (28) and the outlet (6).

2. Device for collecting samples according to claim 1, **characterised in that** the pipe (2) is wider on one side of the nozzle (28) toward the outlet (6) than toward a side of the nozzle toward the vat.

3. Device for collecting samples according to claim 1 or 2, **characterised in that** the nozzle and the valve form a single removable unit with a removable portion (24) of the pipe (2) and a removable portion (25) of the compressed air inlet (26), said removable portions being connected to main portions of the pipe and of the compressed air inlet via capstan connectors (34).

4. Device for collecting samples according to any of claims 1 to 3, **characterised in that** it comprises a plurality of vats (1) and pipes (2), the pipes connecting to each other at a selector (4), and then comprising a joint portion toward the outlet (6), whereon the nozzle (28) and the valve (29) are arranged.

5. Device for collecting samples according to claim 4, **characterised in that** the selector rotates and comprises a cylindrical mobile portion (20) provided with an external member (21) for manoeuvring, the mobile element comprising an axial piercing directed toward the outlet and a radial piercing directed toward the vats and connectors (22) of the pipes arranged on a portion of the selector turn.

6. Device for collecting samples according to any of claims 1 to 5, **characterised in that** the pipe (2) comprises a small reservoir (12), and the sampling jug holder (8) is able to be connected on the small reservoir (12) by the intermediary of a self-sealing quick-coupling in two portions (13, 18), a first of said portions belonging to the small reservoir and a second of said portions comprising a needle (11) which penetrates inside the small reservoir (12) by passing through the first portion.
